# EUROPEAN PATENT APPLICATION

(11) **EP 4 527 835 A1**
(43) Date of publication of application: **26.03.2025**
(21) Application number: 23807495.9
(22) Date of filing: 09.05.2023
(51) Int. Cl.: C07D 331/02

(54) **FLUORENE COMPOUND CONTAINING THIOEPOXY AND (METH)ALLYL GROUPS, AND PRODUCTION METHOD THEREFOR**

(30) Priority: 19.05.2022 JP 2022081983
(71) Applicant: SHIN-ETSU CHEMICAL CO., LTD., Tokyo 1000005 (JP)
(72) Inventor: YANAGISAWA Hideyoshi, Annaka-shi Gunma 379-0224 (JP)
(74) Representative: Mewburn Ellis LLP
(86) International application number: PCT/JP2023/017430
(87) International publication number: WO 2023/223891

(57) **Abstract**

Provided is a fluorene compound represented by formula (1) and containing thioepoxy and (meth)allyl groups. 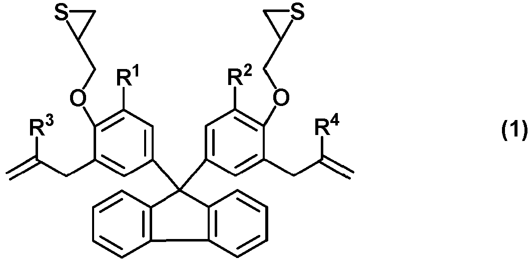 (In the formula, R¹ to R⁴ are each independently a hydrogen atom or a methyl group.)

## Description

### TECHNICAL FIELD

This invention relates to a fluorene compound containing thioepoxy and (meth)allyl groups and a method of preparing the same.

### BACKGROUND ART

One of the fluorene compounds containing (meth)allyl and glycidyl groups known in the art is the compound described in Patent Document 1. Since its refractive index is not high, the compound is insufficient as a raw material for high refractive index optical materials.

### PRIOR ART DOCUMENTS

### PATENT DOCUMENTS

Patent Document 1: JP 4873223

### SUMMARY OF INVENTION

### TECHNICAL PROBLEM

An object of the invention, which has been made under the above-mentioned circumstances, is to provide a compound which can form a polymer having a high refractive index and is thus useful as a raw material for high refractive index optical materials.

### SOLUTION TO PROBLEM

Making extensive investigations to attain the above object, the inventor has found that by reacting a fluorene compound containing glycidyl and (meth)allyl groups, represented by the formula (2): 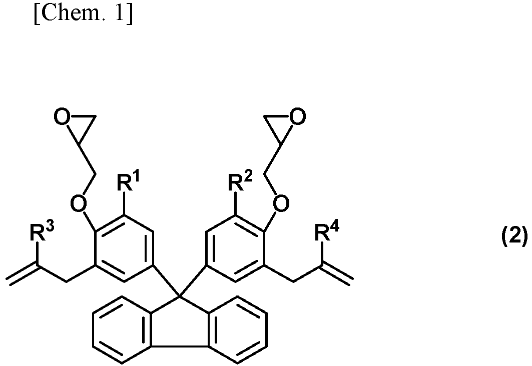
wherein R¹ to R⁴ are each independently hydrogen or methyl with thiourea or a thiocyanate, there is obtained a fluorene compound containing thioepoxy and (meth)allyl groups in the molecule, represented by the formula (1): 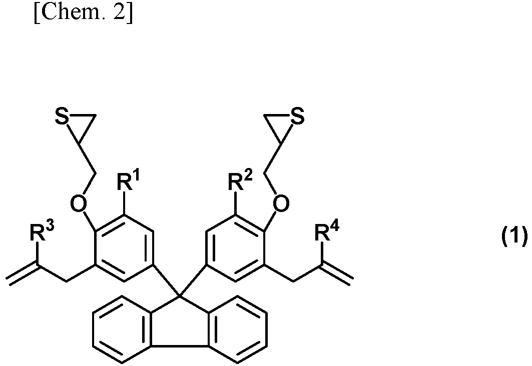
wherein R¹ to R⁴ are each independently hydrogen or methyl. The invention is predicated on this finding.

The invention provides a fluorene compound containing thioepoxy and (meth)allyl groups as defined below.
1. A fluorene compound containing thioepoxy and (meth)allyl groups, represented by the formula (1): 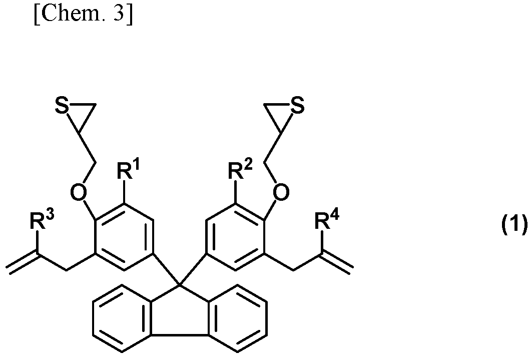 wherein R¹ to R⁴ are each independently hydrogen or methyl.
2. A method of preparing a fluorene compound containing thioepoxy and (meth)allyl groups, represented by the formula (1), the method comprising the step of reacting a fluorene compound containing glycidyl and (meth)allyl groups, represented by the formula (2), with thiourea or a thiocyanate, 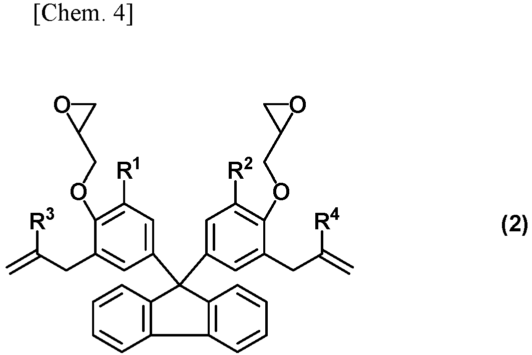
   wherein R¹ to R⁴ are each independently hydrogen or methyl, 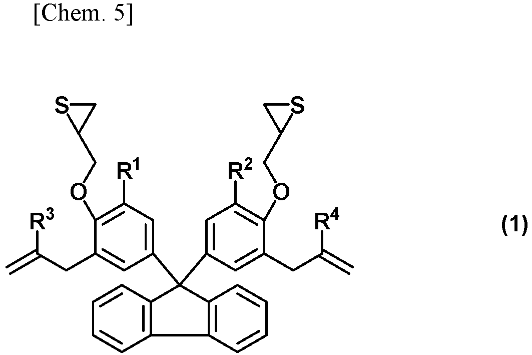
   wherein R¹ to R⁴ are each independently hydrogen or methyl.

### ADVANTAGEOUS EFFECTS OF INVENTION

The fluorene compound containing thioepoxy and (meth)allyl groups according to the invention allows for both polymerization via (meth)allyl groups and polymerization via thioepoxy groups. After polymers are formed, they can be crosslinked and cured by utilizing the groups. Since the fluorene compound containing thioepoxy and (meth)allyl groups according to the invention has two thioepoxy groups in the molecule and aromatic rings capable of achieving a high refractive index, the polymer obtained therefrom is expected to have a high refractive index and is useful as a raw material for high refractive index optical materials.

### DESCRIPTION OF EMBODIMENTS

One embodiment of the invention is a fluorene compound containing thioepoxy and (meth)allyl groups, represented by the formula (1).

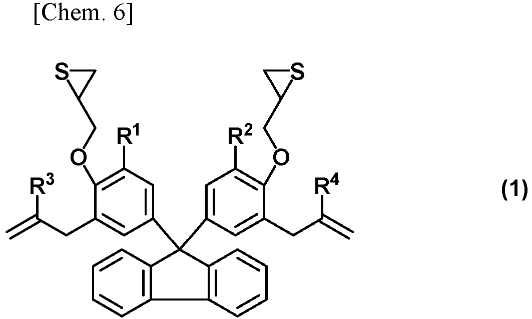

In formula (1), R¹ to R⁴ are each independently hydrogen or methyl. R¹ and R² are preferably hydrogen or methyl, and R³ and R⁴ are preferably hydrogen.

Examples of the compound having formula (1) are shown below, but not limited thereto.

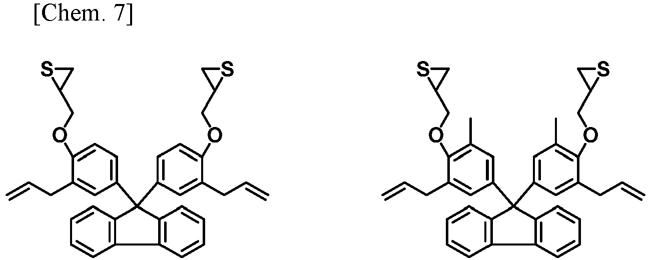

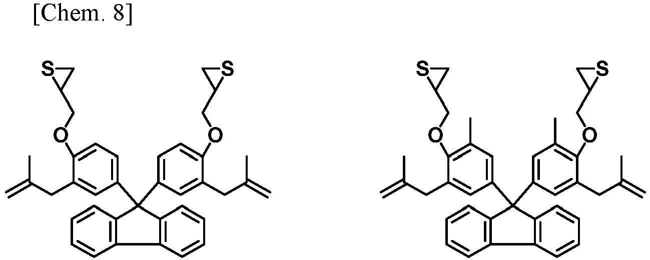

The compound having formula (1) can be prepared by reacting a fluorene compound containing glycidyl and (meth)allyl groups, represented by the formula (2), with thiourea or a thiocyanate.

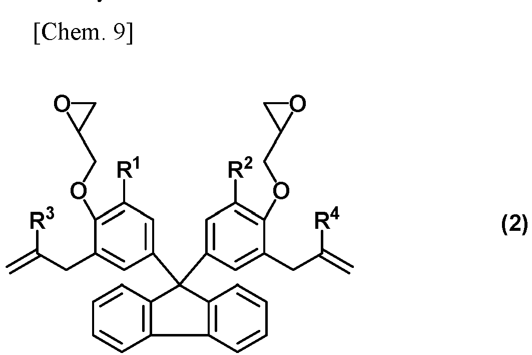

Herein R¹ to R⁴ are as defined above.

Examples of the compound having formula (2) are shown below, but not limited thereto.

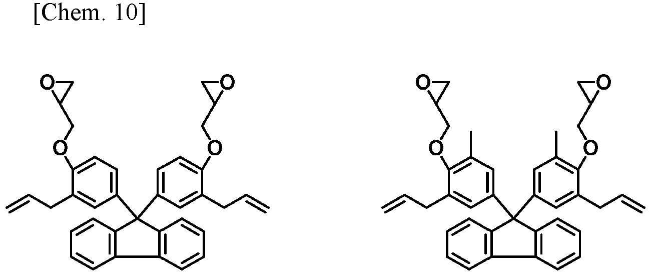

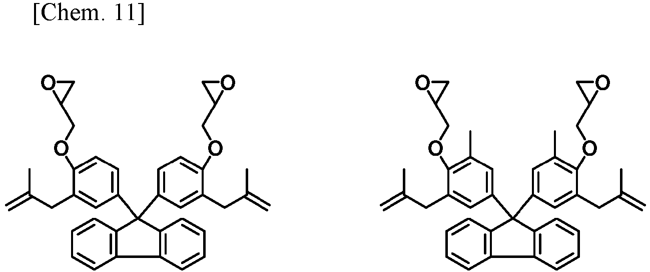

The temperature of the reaction of the compound having formula (2) with a thiocyanate or thiourea is arbitrary, typically 0 to 100°C, preferably 20 to 70°C. The reaction time is not particularly limited as long as the reaction is completed, and is typically 20 hours or less.

The thiocyanate or thiourea is used in a stoichiometric amount corresponding to the stoichiometric amount of epoxy groups of the epoxy compound, but may be used in either smaller amounts or larger amounts when the product purity, reaction rate, economy or the like is taken into account. In the practice of the invention, the thiocyanate or thiourea is preferably used in an amount of more than 0 mole to about 5 moles, more preferably about 0.9 to about 5 moles, even more preferably about 1 to about 3 moles per mole of epoxy groups of the epoxy compound.

The reaction may be performed in a solventless system or in a solvent. When the solvent is used, the solvent in which either of the thiocyanate or thiourea and the compound having formula (2) is soluble is preferably selected. Examples of the solvent include water, alcohols such as methanol and ethanol; ethers such as diethyl ether, tetrahydrofuran and dioxane; hydroxyethers such as methylcellosolve, ethylcellosolve, and butylcellosolve; aromatic hydrocarbons such as benzene, toluene and xylene; and halogenated hydrocarbons such as dichloroethane, chloroform and chlorobenzene. A mixture of these solvents is also acceptable. For example, a mixture of an alcohol with water, or a mixture of an ether, hydroxyether, halogenated hydrocarbon or aromatic hydrocarbon with an alcohol is sometimes quite effective. When the solvent is used, its amount is preferably 0.1 to 500 parts by weight, more preferably 1 to 400 parts by weight per 100 parts by weight of the compound having formula (2).

It is effective to add a polymerization inhibitor to the reaction solution because the reaction result is better. Typical polymerization inhibitors are acids and acid anhydrides. Examples of the acids and acid anhydrides include nitric acid, hydrochloric acid, sulfuric acid, fuming sulfuric acid, boric acid, arsenic acid, phosphoric acid, hydrocyanic acid, acetic acid, peracetic acid, thioacetic acid, oxalic acid, tartaric acid, propionic acid, butyric acid, succinic acid, maleic acid, benzoic acid, nitric anhydride, sulfuric anhydride, boron oxide, diarsenic pentoxide, phosphorus pentoxide, chromic anhydride, acetic anhydride, propionic anhydride, butyric anhydride, succinic anhydride, maleic anhydride, benzoic anhydride, phthalic anhydride, silica gel, silica alumina, and aluminum chloride. When used, the amount of the polymerization inhibitor added is preferably 0.001 to 10% by weight, more preferably 0.01 to 1% by weight of the overall amount of the reaction solution. The polymerization inhibitor may be used alone or in admixture.

By washing the reaction product with an acidic aqueous solution, the resulting compound can be improved in stability. Examples of the acid used in the acidic aqueous solution include nitric acid, hydrochloric acid, sulfuric acid, boric acid, arsenic acid, phosphoric acid, hydrocyanic acid, acetic acid, peracetic acid, thioacetic acid, oxalic acid, tartaric acid, succinic acid, and maleic acid. The acid may be used alone or in admixture. The acidic aqueous solution is often effective at pH 6 or lower, more effective at pH 0 to 3. The acid may be used alone or in admixture.

The fluorene compound containing thioepoxy and (meth)allyl groups is a compound containing two thioepoxy groups and two (meth)allyl groups in the molecule. Using the reactivity of (meth)allyl groups, the fluorene compound can be polymerized through hydrosilation reaction with a siloxane compound having Si-H, obtaining a polymeric silicone material having a structure containing thioepoxy groups and capable of three-dimensional crosslinking. Alternatively, using the reactivity of thioepoxy groups, the fluorene compound can be used as a curable material having a structure capable of three-dimensional crosslinking. After the fluorene compound is converted to a polymer by using thioepoxy or (meth)allyl groups, the polymer can be crosslinked and cured using the remaining functional groups. The fluorene compound containing thioepoxy and (meth)allyl groups is useful as a macromonomer for heat-resistant resin materials having a high refractive index.

### EXAMPLES

Examples of the invention are given below by way of illustration, but not by way of limitation.

### [Example 1]

A 3-L separable flask equipped with a nitrogen gas inlet tube, thermometer, Dimroth condenser, vacuum controller, and aspirator was charged with 271 g (0.5 mol) of a compound having the formula (2-1): 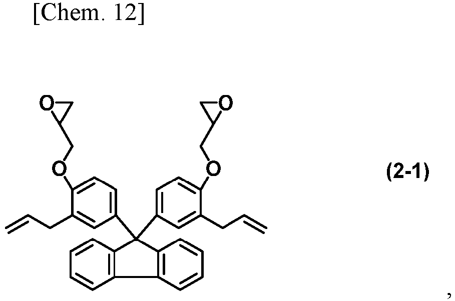 304 g (4 mol) of thiourea, 10 g of acetic anhydride, and 1,000 g of toluene and 1,000 g of methanol as a solvent, which were reacted at 50°C for 8 hours. At the end of reaction, by extracting with toluene, washing with 1 wt% sulfuric acid, washing with water, and removing the excess solvent, 272 g of the product was obtained. On elemental analysis by automatic elemental analyzer 2400II CHNS/O (Perkin Elmer), GC-MS mass analysis by analyzer 7890A (Agilent Technologies), ¹H-NMR spectroscopy by analyzer AV400M (Bruker Corp.), and FT-IR spectroscopy by Nicolet 6700 (Thermo Fisher Scientific), the product was found to be a compound having the following formula (1-1). Yield 95%. Toluene, 30 g, was added to 70 g of the compound having formula (1-1) to form a 70 wt% toluene solution, which was measured for refractive index by Abbe Refractometer NAR-1T LIQUID (Atago Co., Ltd.). A refractive index of 1.58 was found, indicating a highly refractive compound.

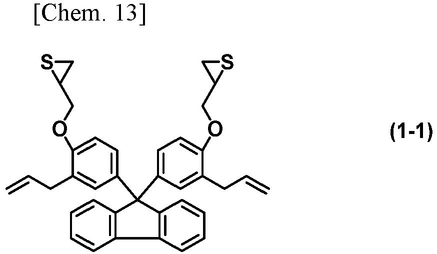

### [Example 2]

By following the same procedure as in Example 1 aside from using 285 g (0.5 mol) of a compound having the formula (2-2): 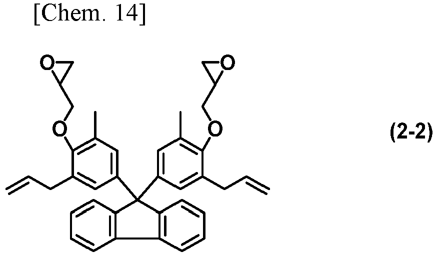 instead of the compound having formula (2-1), there was obtained 288 g of the product. On elemental analysis by automatic elemental analyzer 2400II CHNS/O (Perkin Elmer), GC-MS mass analysis by analyzer 7890A (Agilent Technologies), ¹H-NMR spectroscopy by analyzer AV400M (Bruker Corp.), and FT-IR spectroscopy by Nicolet 6700 (Thermo Fisher Scientific), the product was found to be a compound having the following formula (1-2). Yield 96%. Toluene, 30 g, was added to 70 g of the compound having formula (1-2) to form a 70 wt% toluene solution, which was measured for refractive index by Abbe Refractometer NAR-1T LIQUID (Atago Co., Ltd.). A refractive index of 1.58 was found, indicating a highly refractive compound.

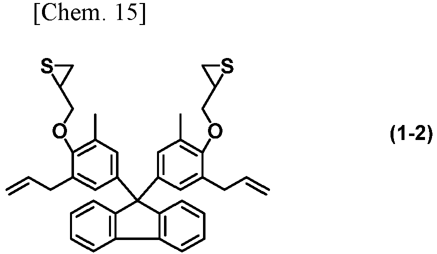

## Claims

1. A fluorene compound containing thioepoxy and (meth)allyl groups, represented by the formula (1): 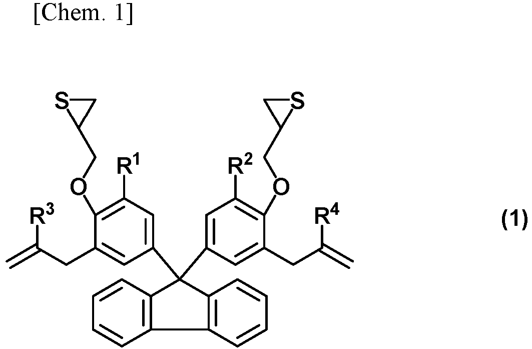 wherein R¹ to R⁴ are each independently hydrogen or methyl.

2. A method of preparing a fluorene compound containing thioepoxy and (meth)allyl groups, represented by the formula (1), the method comprising the step of reacting a fluorene compound containing glycidyl and (meth)allyl groups, represented by the formula (2), with thiourea or a thiocyanate, 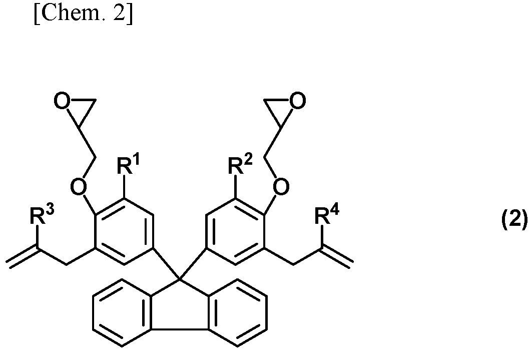
wherein R¹ to R⁴ are each independently hydrogen or methyl, 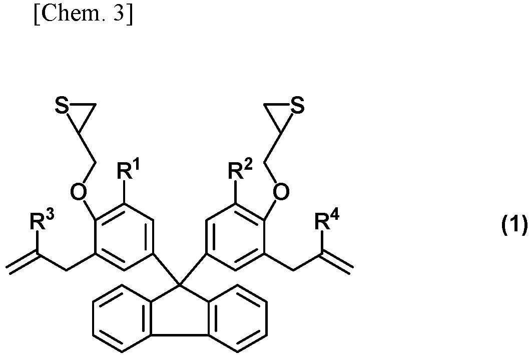
wherein R¹ to R⁴ are each independently hydrogen or methyl.
